# EUROPÄISCHE PATENTSCHRIFT

(11) **EP 0 937 452 B1**
(45) Veröffentlichungstag und Bekanntmachung des Hinweises auf die Patenterteilung: **27.03.2002**
(21) Anmeldenummer: 98124166.4
(22) Anmeldetag: 19.12.1998
(51) Int. Cl.: A61K 7/13, A61K 7/08, A61K 7/50

(54) **Verwendung von polyquaternären Ammoniumverbindungen in Haarbehandlungsmitteln**
Use of polyquaternary ammonium compounds in haircare compositions
Utilisation des composés d' ammonium polyquaternaire dans des produits pour le traitement du cheveux

(30) Priorität: 31.12.1997 DE 19758272
(43) Veröffentlichungstag der Anmeldung: 25.08.1999
(73) Patentinhaber: GOLDWELL GmbH, 64297 Darmstadt (DE)
(72) Erfinder: Grit, Mustafa, Dr., 64579 Gernsheim (DE)

(56) Entgegenhaltungen:
- EP-A- 0 902 009
- WO-A-97/16516
- WO-A-99/11224
- DE-A- 19 622 815
- DATABASE CHEMICAL ABSTRACTS [Online] STN Zusamenfassung 124: 319 622, XP002136394 & JP 08 027669 A (LION CORP.) 30. Januar 1996 (1996-01-30)

## Beschreibung

Die vorliegende Erfindung betrifft die Verwendung von anionischen UV-Absorbern und/oder direktziehenden anionischen Farbstoffen in einem Haarbehandlungsmittel mit verbesserten Gebrauchseigenschaften, insbesondere verbessertem Lichtschutz bzw. einer verbesserten Färbeintensität und Farbstabilität.

Es ist bereits seit längerem bekannt, Haarbehandlungsmitteln, insbesondere Haarnachbehandlungsmitteln im Sinne der Definition in der Monographie von K. Schrader, Grundlagen und Rezepturen der Kosmetika, 2. Auflage (1989, Hüthig Buch Verlag), S. 722-724, UV-Absorber, beispielsweise mit anionischen Gruppen, zuzusetzen, um die Haare gegen schädlichen Lichteinfluß zu schützen.
Je stärker diese Verbindungen auf das Haar aufziehen, desto besser logischerweise der erzielte Lichtschutz.
Dieser ist jedoch oftmals nicht optimal, da beispielsweise durch verschiedene Bestandteile von Haarbehandlungsmitteln das Aufziehen dieser UV-Absorber ver- oder zumindest behindert wird.
Gleiches gilt hinsichtlich anionischer direktziehender Haarfarbstoffe, weshalb in der Regel in Haartönungsmitteln nur kationische direktziehende Haarfarbstoffe eingesetzt werden.
Dies gilt natürlich auch für Zusammensetzungen, die beide Substanzgruppen, nämlich sowohl UV-Absorber als auch anionische direktziehende Haarfarbstoffe enthalten, was insofern für die Praxis von Bedeutung ist, als durch die gleichzeitige Verwendung von Direktziehern und UV-Absorbern die vorzeitige Ausbleichung der erzielten Haarfärbung durch Lichteinfluß verhindert werden kann.
Die Erfindung geht daher von der Aufgabenstellung aus, ein Haarbehandlungsmittel, das anionische UV-Absorber und/oder anionische direktziehende Haarfarbstoffe enthält, zu schaffen, das die erwähnten Nachteile nicht aufweist, sondern einen stabilen Lichtschutz und eine dauerhafte Haarfärbung gewährleistet.

Diese Aufgabe wird dadurch gelöst, daß einem Haarbehandlungsmittel auf wäßriger Basis, das mindestens 0,05 bis 5 Gew.-% eines anionisches UV-Absorbers und/oder 0,01 bis 2,5 Gew.-% mindestens einen anionischen Haarfarbstoff enthält, 0,5 bis 20 Gew.-% mindestens einer Verbindung der allgemeinen Formel I 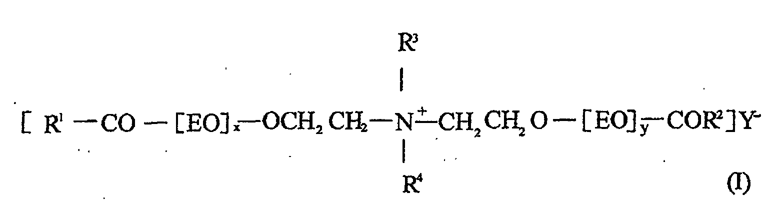 in der R¹ und R² jeweils für eine gegebenenfalls hydroxysubstituierte C₈-C₂₂- Alkyl- oder Alkenylgruppe, R³ und R⁴ für eine C₁-C₃-Alkylgruppe oder eine Gruppe -CH₂-CH₂-O-[EO]_{z}-H, sowie x,y und z für 0 bis 5, und Y⁻ für ein Anion stehen, wobei die Menge jeweils auf die Gesamtzusammensetzung des Mittels bezogen sind, zur Abscheidung des anionischen UV-Absorbers und des anionischen Haarfarbstoffes auf menschliches Haar zugesetzt werden.

Bevorzugte Reste R¹ und R² sind C₁₂-C₁₈-Alkyl- und Oleylreste, der Rest R³ eine Methylgruppe und der Rest R⁴ eine Hydroxyethylgruppe, x,y und z sind vorzugsweise 0 oder 1; Y⁻ ist vorzugsweise ein Methosulfat-, Chlorid- oder Phosphatanion.

Die Menge an dieser Verbindung der Formel I liegt insbesondere bei 0,5 bis 15, vor allem bei 1 bis 10 Gew.-%, berechnet auf das Haarbehandlungsmittel.

Diese Verbindungen sind an sich bekannt und beispielsweise unter den Markenbezeichnungen "Schercoquat^{R}", Dehyquart^{R}F30" und "Tetranyl^{R}" im Handel.

Bei den erfindungsgemäß verwendeten Haarbehandungmitteln handelt es sich entweder um Tönungsshampoos auf Basis anionischer direktziehender Haarfarbstoffe oder um Haarnachbehandlungsmittel, die diese Farbstoffe und/oder anionische UV-Absorber enthalten.

Letztere werden in einer Menge von 0,05 bis 5, insbesondere 0,1 bis 2,5 Gew.-%, berechnet auf die Gesamtzusammensetzung des Mittels, eingesetzt.
Durch die Verwendung dieser Zusammensetzung wird ein stabiler Lichtschutz erreicht.

Besonders geeignete wasserlösliche UV-Absorber mit anionischen Gruppen sind beispielsweise 5-Benzoyl-4-hydroxy-2-methoxybenzolsulfonsäure (Benzophenone-4), dessen Natriumsalz (Benzophenone-5) und 2,2'-Dihydroxy-4,4'-dimethoxy-3,3'-disulfobenzophenon bzw. dessen Dinatriumsalz (Benzophenone-9) sowie Phenylbenzimidazolsulfonsäure (Eusolex® 232); jedoch können auch andere anionische UV-Absorber eingesetzt werden.

Der Anteil der direktziehenden anionischen Haarfarbstoffe in den erfindungsgemäß verwendeten Zusammensetzungen ist variabel und liegt zwischen 0,01 bis 2,5, vorzugsweise 0,01 bis 1, insbesondere 0,1 bis 0,5 Gew.-% des Mittels, falls es als Lösung, Dispersion, Emulsion, Gel oder Aeroslpräparat zur direkten Anwendung, d.h., ohne vorherige Verdünnung vorliegt.
Bei Konzentraten, die vor der Anwendung verdünnt werden, ist der Anteil natürlich entsprechend höher.
Dadurch wird eine besonders dauerhafte Haarfärbung erreicht.
Als geeignete anionische Farbstoffe können Verwendung finden:
- Acid Black 1,: C.I.-No. 20,470;
- Acid Blue 1,: C.I.-No. 42,045;
- Food Blue 5,: C.I.-No. 42,051;
- Acid Blue 9,: C.I.-No. 42,090;
- Acid Blue 74,: C.I.-No. 73,015;
- Acid Red 18,: C.I.-No. 16,255;
- Acid Red 27,: C.I.-No. 16,185;
- Acid Red 87,: C.I.-No. 45,380;
- Acid red 92,: C.I.-No. 45,410;
- Acid Violet 43,: C.I.-No. 60,730;
- Acid Yellow 1,: C.I.-No. 10,3 16;
- Acid Yellow 23,: C.I.-No. 19,140;
- Acid Yellow 3,: C.I.-No. 47,005;
- Food Yellow No. 8,: C.I.-No. 14,270;
- D&C Brown No. 1,: C.I.-No. 20,170
- D&C Green No. 5,: C.I.-No. 61,570;
- D&C Orange No. 4,: C.I.-No. 15,510;
- D&C Orange No. 10,: C.i.-No 45,425:1;
- D&C Orange No. 11,: C.I.-No. 45,425;
- D&C Red No. 21,: C.I.-No. 45,380:2;
- D&C Red No. 27,: C.L-No. 45,410:1;
- D&C Red No. 33,: C.I.-No. 17,200;
- D&C Yellow No. 7,: C.I.-No. 45,350:1;
- D&C Yellow No. 8,: C.I.-No. 45,350;
- FD&C Red No. 4,: C.I.-No. 14,700;
- FD&C Yellow No. 6,: C.I.-No. 15,985.

Auch pflanzliche Farbstoffe können, wenn sie anionisch sind, allein, oder falls sie nicht anionisch sind, in Kombination mit anionischen synthetischen Direktziehern Verwendung finden, beispielsweise Henna (rot oder schwarz), Alkannawurzerl, Laccainsäure (Stocklack), Blauholzpulver, Krappwurzel- und Rhabarberwurzelpulver, etc.

Bei den erfindungsgemäß verwendeten anionische UV-Absorber enthaltenden Mitteln kann es sich auch um Färbemittel handeln, die zusätzlich kationische direktziehende Farbstoffe und Verbindungen der Formel I enthalten; diese führen aufgrund des besseren Lichtschutzes zu dauerhaften Färbungen.

Die erfindungsgemäß verwendeten Zusammensetzungen enthalten, insbesondere wenn es sich um Haarnachbehandlungsmittel im Sinne der eingangs genannten Definition handelt, vorzugsweise noch mindestens ein kationisches Tensid, insbesondere in einer Menge von 0,1 bis 7,5, vorzugsweise 0,25 bis 5, besonders bevorzugt 0,5 bis 2,5 Gew.-%, der Gesamtzusammensetzung.

Geeignete langkettige quaternäre Ammoniumverbindungen, die als kationische Tenside allein oder im Gemisch miteinander eingesetzt werden können, sind insbesondere Cetyltrimethylammoniumchlorid, Dimethyldicetylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Lauryltrimethylammoniumchlorid, Tris(oligooxyethyl)alkylammoniumphosphat, Cetylpyridiniumchlorid, Behentrimoniumchlorid, etc.
Gut geeignet sind auch die in der EP-A 472 107 geoffenbarten quaternären Ammoniumsalze. Im Prinzip sind alle quaternären Ammoniumverbindungen, wie sie im "CTFA International Cosmetic Ingredient Dictionary", Fourth Ed. (1991), unter dem Trivialnamen "Quaternium" aufgeführt sind, geeignet.

Die Zusammensetzung kann natürlich zusätzlich die in solchen Mitteln üblichen Bestandteile enthalten; es wird, zur Vermeidung von Wiederholungen, auf K.Schrader, S.722 - 771, verwiesen.

Auch nichtionische Tenside können, insbesondere im Gemisch mit kationaktiven Tensiden, Verwendung finden, beispielsweise Aminoxide in einer Menge von 0,25 bis 5, vorzugsweise 0,5 bis 3,5 Gew.-%, berechnet auf die Gesamtzusammensetzung des Mittels.

Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl- oder -ethylaminoxide, C₁₂-C₁₈-Alkyldi(hydroxyetyl)- oder -(hydroxypropyl)aminoxide, oder auch Aminoxide mit Ethylenoxid- und /oder Propylenoxidgruppen in der Alkylkette.

Geeignete Tenside sind weiterhin die bekannten C₈-C₁₈-Alkylpolyglucoside, insbesondere mit einem Polykondensationsgrad von 1,2 bis 3.

Wenn es sich bei den erfindungsgemäß verwendeten Zusammensetzungen um Tönungsshampoos handelt, können auch anionische Tenside, vorzugsweise im Gemisch mit nichtionischen, amphoteren und/oder zwitterionischen Tensiden, eingesetzt werden.

Geeignete anionische Tenside sind natürlich diejenigen, die in Shampoos üblicherweise zum Einsatz gelangen, beispielsweise die bekannten C₁₀-C₁₈-Alkylsulfate und insbesondere die entsprechenden Ethersulfate, beispielsweise C₁₂-C₁₄-Alkylethersulfat, Laurylethersulfat, insbesondere mit 1 bis 4 Ethylenoxidgruppen im Molekül, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettiger Mono- und Dialkylphosphate, die milde, hautverträgliche Detergentien darstellen.

Im Rahmen der Erfindung weiterhin geeignete anionische Tenside sind auch Isethionate, α-Olefinsulfonate bzw. deren Salze und insbesondere Alkalisalze von Sulfobernsteinsäurehalbestern, beispielsweise das Dinatriumsalz des Monooctylsulfosuccinats und Alkalisalze langkettiger Monoalkylethoxysulfosuccinate.

Geeignete Tenside vom Carboxylat-Typ sind Alkylpolyethercarbonsäuren und deren Salze der Formel

R - (C₂H₄O)ₙ - O - CH₂ COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel 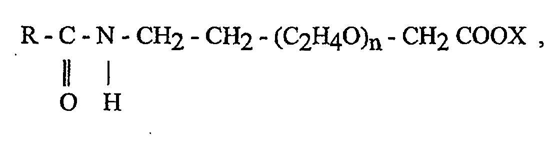 worin R und X die vorstehend angegebene Bedeutung haben und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO" und "AKYPO-SOFT®".

Es ist besonders zweckmäßig, Mischungen aus mehreren anionischen Tensiden einzusetzen, beispielsweise ein Gemisch aus einem α-Olefinsulfonat und einem Sulfosuccinat, vorzugsweise im Verhältnis von 1 : 3 bis 3 : 1, oder einem Ethersulfat und einer Polyethercarbonsäure oder Alkylamidoethercarbonsäure.

Im Gemisch mit anderen anionischen Tensiden ebenfalls einsetzbar sind Eiweiß-Fettsäure-Kondensationsprodukte an sich bekannter Struktur, insbesondere in Mengen zwischen 0,5 und 5, vorzugsweise 1 bis 3 Gew.-% der Gesamtzusammensetzung des flüssigen Haarwaschmittels.

Eine Übersicht über die in flüssigen Haarbehandlungsmitteln zum Einsatz gelangenden anionaktiven Tenside findet sich im übrigen in der Monographie von K.Schrader, S. 683 bis 691.

Der bevorzugte Mengenbereich an anionischen Tensiden in den erfindungsgemäß verwendeten flüssigen Haarbehandlungsmitteln liegt zwischen 2,5 und 2,5 Gew.-%, insbesondere bei 5 bis 20 Gew.-%, besonders bevorzugt bei 7,5 bis 15 Gew.-%, berechnet auf die Gesamtzusammensetzung des Mittels.

Nichtionische Tenside gelangen in Tönungsshampoos vorzugsweise im Gemisch mit anionaktiven Tensiden zum Einsatz.

Ein bevorzugtes nichtionisches Tensid gehört dabei, wie bereits oben erwähnt, zu der Klasse der Alkylpolyglucoside der allgemeinen Formel

R - O - (R¹O)ₙ - Zₓ ,

worin R eine Alkylgruppe mit 8 bis 18 Kohlenstoffatomen, R¹ eine Ethylen- oder Propylengruppe, Z einen Saccharidrest mit 5 bis 6 Kohlenstoffatomen, n eine Zahl von 0 bis 10 und x eine Zahl zwischen 1 und 2,5 bedeuten.
Diese Alkylpolyglucoside sind in letzter Zeit insbesondere als ausgezeichnete hautverträgliche schaumverbessernde Mittel in flüssigen Wasch- und Körperreinigungsmitteln bekannt geworden und sind vorzugsweise in einer Menge von 1 bis 20, insbesondere 2,5 bis 15 Gew.-%, der Gesamtzusammensetzung enthalten.

Weitere nichtionische Tensidbestandteile in Tönungsshampoos sind beispielsweise langkettige Fettsäuremono- und -dialkanolamide, beispielsweise Cocosfettsäuremonoethanolamid und Myristinfettsäuremonoethanolamid, die auch als Schaumverstärker eingesetzt werden können.

Andere nichtionische Tenside sind beispielsweise die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind.

Gemische aus anionaktiven Tensiden und Alkylpolyglucosiden, den bevorzugten nichtionischen Tensiden im Rahmen der Erfindung sowie deren Verwendung in flüssigen Körperreinigungsmitteln sind an sich bereits bekannt, beispielsweise aus der EP-A 70 074. Die dort beschriebenen Alkylpolyglucoside sind prinzipiell auch im Rahmen der vorliegenden Erfindung geeignet; ebenso die aus der EP-A 358 216 bekannten Gemische aus Sulfosuccinaten und Alkylpolyglucosiden.

Weitere im Gemisch mit anionaktiven Tensiden einsetzbare Tenside sind die bereits erwähnten Aminoxide in einer Menge von 0,25 bis 5, vorzugsweise 0,5 bis 3,5 Gew.-%, berechnet auf die Gesamtzusammensetzung des Mittels.

Als amphotere bzw. zwitterionische Tenside können beispielsweise Betaine, Sulfobetaine und/oder Alkylaminocarbonsäuren wie Alkylaminoglycinate, Alkylaminopropionate, Alkylglycinate, etc. eingesetzt werden. Besonders geeignet sind Alkylamidobetaine der allgemeinen Formel 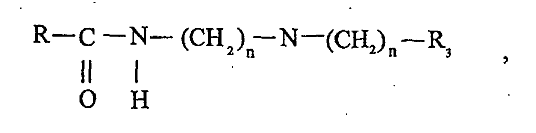 wobei R eine C₈-C₁₈-Alkylgruppe, z. B. einen Cocoalkylrest; R₁ und R₂ einen niederen C₁-C₄-Alkyl- oder -Hydroxyalkylrest, insbesondere eine Methyl-, Ethyl- und/oder Hydroxyethylgruppe; R₃ eine COO⁻-oder -SO₃⁻-Gruppe; und n 1 bis 3 bedeuten.

Auch Betaine der allgemeinen Formel 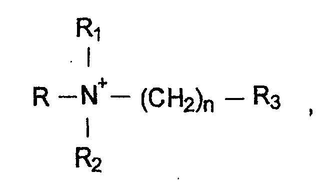 wobei R, R₁, R₂, R₃ und n die obengenannte Bedeutung haben, sind bevorzugt.

Geeignet sind insbesondere auch Handelsprodukte wie beispielsweise "Tegobetaine® ", "Dehytone® " wie "AB 30", "G" und "K", "Lonzaine® ", "Varion® " wie "ADG" und "CAS", "Lexaine®", "Chembetaine® ", "Mirataine® ", "Rewoteric® ", "Schercotaine® ", "Monteine LCQ® "; ""Alkateric® ", "Amonyl® ", "Amphosol® ", "Cycloteric BET® ", "Emcol® ", "Empigen® ", "Mackam® ", "Monateric® ", "Unibetaine® " und "Velvetex® ".

Die amphoteren bzw. zwitterionischen Tenside sind vorzugsweise in einer Menge von 0,25 bis 7,5 Gew.-%, insbesondere 0,5 bis 5 Gew.-%, berechnet auf die Gesamtzusammensetzung, enthalten.

Die erfindungsgemäß verwendeten Haarbehandlungsmittel können die in solchen wäßrigen Zubereitungen üblichen Stoffe enthalten.

Dies sind beispielsweise synthetische oder natürliche haarkonditionierende Polymere, vorzugsweise in einer Menge von 0,1 bis 2,5, insbesondere 0,25 bis 1,5 Gew.-% der Gesamtzusammensetzung.

Als geeignete kationische Polymere sind neben den altbekannten quaternären Cellulosederivaten des Typs "Polymer JR" insbesondere quaternisierte Homo- und Copolymere des Dimethyldiallylammoniumchlorids, wie sie unter dem Handelsnamen "Merquat" im Handel sind, quaternäre Vinylpyrrolidon-Copolymere, insbesondere mit Dialkylaminoalkyl(meth)-acrylaten, wie sie unter dem Namen "Gafquat" bekannt sind, Copolymerisate aus Vinylpyrrolidon und Vinylimidazoliniummethochlorid, die unter dem Handelsnamen "Luviquat" angeboten werden, Polyamino-Polyamid-Derivate, beispielsweise Copolymere von Adipinsäure-Dimethylaminohydroxypropyldiethylentriamin, wie sie unter dem Namen "Cartaretine F" vertrieben werden, sowie auch bisquaternäre langkettige Ammoniumverbindungen der in der US-PS 4 157 388 beschriebenen Harnstoff-Struktur, die unter dem Handelsnamen "Mirapol A 15" im Handel sind, geeignet.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.

Anstelle der kationischen Polymeren oder in Kombination mit denselben können auch nichtionische Polymere verwendet werden. Als geeignete nichtionische Polymere werden vor allem Vinylpyrrolidon-Homo- und Copolymerisate, insbesondere Polyvinylpyrrolidon selbst, Copolymere aus Vinylpyrrolidon und Vinylacetat oder Terpolymerisate aus Vinylpyrrolidon, Vinylacetat und Vinylpropionat, wie sie beispielsweise von der Firma BASF unter dem Handelsnamen "Luviskol" vertrieben werden, eingesetzt.

Es können jedoch auch (Co-)Polymerisate aus den verschiedenen Acryl- und Methacrylestern, Acrylamid und Methacrylamid, beispielsweise Polyacrylamid mit Molgewichten von über 100.000, Dimethylhydantoin-Formaldehyd-Harze, etc., verwendet werden. Selbstverständlich sind auch Mischungen aus verschiedenen nichtionischen Polymeren verwendbar.

Geeignet sind schließlich auch noch amphotere Polymere, z. B. die unter der Bezeichnung "Amphomer" vertriebenen Copolymerisate aus N-Octylacrylamid, N-Butylaminoethylmethacrylat und Acrylsäure, sowie die bekannten anionischen Produkte, die im Stand der Technik hinreichend beschrieben sind.

Die erfindungsgemäß verwendeten Haarbehandlungsmittel können, wenn sie als Emulsionen, Dispersionen, Lösungen, Gele oder Aerosole als Nachbehandlungsmittel eingesetzt werden, die in solchen Zusammensetzungen üblichen Zusätze enthalten, deren Art und Charakter von der Applikationsform des Mittels abhängig ist. Es sind dies Fette, Fettalkohole, Emulgatoren, pH-Regulatoren, Lösungs- und Verdünnungsmittel, Lösungsvermittler, Konservierungsmittel, Parfums, etc.

Geeignete Fette und Öle, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.

Synthetische Öle und Wachse sind beispielsweise Silikonöle und Polyethylenglykole.

Weitere geeignete hydrophobe Komponenten sind insbesondere Fettalkohole, vorzugsweise solche mit 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat und Cetylpalmitat.
Diese hydrophoben Komponenten sind in den erfindungsgemäß verwendeten Zusammensetzungen vorzugsweise in einer Gesamtmenge von 0,5 bis 10, insbesondere 1 bis 7,5, vor allem 1,5 bis 5 Gew.-%, berechnet auf die Gesamtzusammensetzung, enthalten.

Eine bevorzugte Gruppe von aktiven Ingredientien sind C₁₀-C₂₄-Fettsäuren, insbesondere Behensäure und Stearinsäure, vorzugsweise zwischen 0,1 und 10 Gew.-% der Gesamtzusammensetzung.
Eine Zusammenfassung der Herstellung solcher Mittel findet sich in der bereits erwähnten Monographie von K. Schrader, S. 798 bis 815, insbesondere S. 804 ff.

Die erfindungsgemäß verwendeten Haarbehandlungmittel liegen als Emulsion, Dispersion oder (gegebenenfalls verdickte, d. h. als Gel) Lösung vor und können auch als Aerosolschaum konfektioniert werden. Diese Zusammensetzungen und ihre Herstellung sind dem Fachmann grundsätzlich bekannt und bedürfen daher keiner näheren Erläuterung.

Der pH-Wert der erfindungsgemäß verwendeten Haarfärbemittel liegt vorzugsweise bei 3 bis 8, insbesondere zwischen 4 und 6.

Aus der prioritätsälteren, nicht vorveröffentlichten WO 99/11224 A ist die Verwendung von Kombinationen aus einem faserstrukturverbessernden Wirkstoff A, insbesondere Panthenol und dessen Derivaten, Pflanzenextrakten und Honig, und einem konditionierenden Wirkstoff B, insbesondere Esterquats und Amidoamine, bekannt.

Das Abstract der JP 08 027669 A beschreibt lagerstabile Textilbehandlungsmittel, die 3 bis 30 Gew.-% Textilweichmacher (Esterquat oder Amidoquat), 1 bis 100 ppm Acid Yellow 3 und/oder Acid Yellow 141 und 0,1 bis 1000 ppm 2,6-Di-tert.-Butyl-4-methylphenol enthalten.

Die DE 196 22 815 A1 schließlich offenbart Haarbehandlungsmittel mit verbesserten haarkonditionierenden Eigenschaften mit einem pH-Wert von 1,5 bis 6, die 0,1 bis 8 Gew.-% eines Difettsäuredialkanolaminestersalzes als konditionierenden Wirkstoff enthalten und frei von kationischen Tensiden sind.

Die folgenden Beispiele beschreiben Zusammensetzungen der erfindungsgemäßen Mittel. Es wurden Nachbehandlungsmittel bzw. Tönungsshampoos der folgenden Zusammensetzungen durch Vermischen der Bestandteile, gegebenenfalls in Form von Vormischungen, hergestellt.

### Beispiele 1 bis 4

| **Haarkonditioner mit Lichtschutz** | | | | |
|---|---|---|---|---|
| | **1** | **2** | **3** | **4** |
| Cetylstearylalkohol | 5,00 | 2,00 | 5,00 | 3,00 |
| Mineralöl | 0,50 | 0,50 | 0,50 | 0,50 |
| Isopropylmyristat | 0,50 | 0,50 | 0,50 | 0,50 |
| Hydroxyethylcellulose | - | 1,00 | - | 1,00 |
| Benzophenone-5 | - | 0,50 | - | - |
| Benzophenone-4 | 0,30 | - | 0,30 | - |
| Benzophenone-9 | - | - | - | 0,80 |
| Verbindung der Formel I* | 1,50 | 1,00 | 2,50 | 1,50 |
| Cocamidopropylbetain | - | - | 0,50 | 0,50 |
| Basic Brown 17 | - | - | 0,10 | 0,10 |
| Basic Brown 16 | - | - | 0,05 | 0,05 |
| Basic Blue 99 | - | - | 0,03 | 0,03 |
| Parfum | 0,20 | 0,20 | 0,20 | 0,20 |
| Konservierungsmittel | q.s. | q.s. | q.s. | q.s. |
| Wasser | ad 100,00 | ad 100,00 | ad 100,00 | ad 100,00 |

| | | | | |
|---|---|---|---|---|
| *Beispiele 1 und 3: R¹=R²=Oleyl; R³=CH₃;R⁴=CH₂CH₂OH;x=y=0; Y⁻=CH₃S0⁻₄. Beispiel 2: R¹=R²=C₁₈H₃₇; R³=CH₃; R⁴=CH₂CH₂OH; x=y=0; Y⁻=Cl⁻ Beispiel 4: R¹=R²=C₁₂H₂₅; R³=CH₃; R⁴=CH₂CH₂OH; x=y=0; Y⁻=Cl⁻ | | | | |

### Vergleichstests

a) Eine Zusammensetzung 1 nach Beispiel 1 wurde auf Haarsträhnen aufgebracht und im Vergleich zu anderen Haarsträhnen, die mit einer ansosnsten identischen Zusammensetzung 1A, die anstelle der Verbindung der Formel I den gleichen Anteil Cetyltrimethylammoniumchlorid enthielt, behandelt worden waren, der Gehalt an UV-Absorber in Haarproben nach mehreren Haarwäschen bestimmt.

Dabei zeigte sich, daß bereits der Ausgangswert an UV-Absorbern in den mit der Zusammensetzung 1 behandelten Strähnen signifikant höher war als in den mit der Zusammensetzung 1A behandelten Strähnen; nach 3 Haarwäschen betrug die Konzentration an UV-Absorber für die erfindungsgemäß verwendete Zusammensetzung etwa das Doppelte der mit der Vergleichszusammensetzung erzielten.

b) Eine Zusammensetzung 3 nach Beispiel 3 wurde auf menschliches Haar aufgebracht, wobei eine glänzende, intensive, ausgeprägte Braunfärbung erzielt wurde, die auch nach 3 Haarwäschen noch stabil war.

Die Färbung mit einer ansonsten identischen Zusammensetzung 3A, die anstelle einer Verbindung der Formel I die gleiche Menge Cetylstearyltrimethylammoniumchlorid enthielt, war merklich matter und nach dreimaliger Haarwäsche weitgehend verblaßt.

### Beispiel 5

| **Haarspülung** | |
|---|---|
| 1,2-Propylenglykol | 2,0 (Gew.-%) |
| Decylpolyglucosid | 1,5 |
| Verbindung der Formel I | 1,0 |
| (R¹=R²=Oleyl; R³=CH₃; R⁴=CH₂CH₂OH; | |
| x=y=0; Y⁻=CH₃SO₄⁻) | |
| Dimethicone Copolyol Bienenwachs | 1,0 |
| Cetylstearylalkohol | 1,0 |
| Hydroxyethylcellulose | 0,8 |
| Kamillenextrakt | 0,3 |
| Benzophenone-4 | 0,2 |
| Behentrimoniumchlorid | 0,5 |
| Parfum | 0,2 |
| Methylparaben | 0,2 |
| Pyrrolidoncarbonsäure | 0,1 |
| Citronensäure | 0,1 |
| Wasser | ad 100,0 |

Die Zusammensetzung weist auf dem Haar ausgezeichnete Lichtschutzeigenschaften auf.

### Beispiele 6 bis 9

| **Haarfärbemittel** | | | | |
|---|---|---|---|---|
| | **6** | **7** | **8** | **9** |
| Cetylstearylalkohol | 5,00 | 5,00 | 3,00 | 3,00 |
| Verbindung der Formel I * | 2,00 | 1,00 | 2,00 | 1,00 |
| Hydroxyethylcellulose | - | - | 1,00 | 1,00 |
| Cetrimoniumchlorid | - | 2,00 | - | 2,00 |
| D.C. Red 33 | 0,10 | 0,08 | 0,10 | 0,10 |
| Acid Yellow 3 | - | 0,02 | - | - |
| FDC-Yellow 6 | - | 0,05 | 0,05 | 0,05 |
| Food Blue 5 | - | 0,05 | - | 0,03 |
| Food Yellow 8 | - | 0,05 | 0,02 | - |
| Parfum | 0,20 | 0,20 | 0,20 | 0,20 |
| Konservierungsmittel | q.s. | q.s. | q.s. | q.s. |
| Wasser | ad 100,00 | ad 100,00 | ad 100,00 | ad 100,00 |
| Erzielter Farbton | Rot | Braun | Rotblond | Hellbraun |

| | | | | |
|---|---|---|---|---|
| * Beispiele 6 und 7 R¹=R²=Oleyl; R³=CH₃;R⁴=CH₂CH₂OH;x=y=0;Y⁻=CH₃S0⁻₄. In den Beispielen 8 und 9 war R¹=R²=C₁₈H₃₇; R³=CH₃;R⁴=CH₂-CH₂-OH; x=y=0; Y⁻=Cl⁻. | | | | |

Die erzielten Färbungen erwiesen sich als wesentlich beständiger als diejenigen, die mit identischen Zusammensetzungen erzielt wurden, die keine Verbindung der Formel I und statt dessen einen entsprechend erhöhten Anteil an Cetrimoniumchlorid enthielten.

### Beispiele 10 bis 14

| **Tönungsshampoo** | | | | | |
|---|---|---|---|---|---|
| **Beispiel Nr.** | **10** | **11** | **12** | **13** | **14** |
| Natrium-C₁₂-C₁₄-alkylethersulfat (~2-3EO-Gruppen) | 10,00 | - | 8,00 | 10,00 | - |
| C₁₂-C₁₄-Alkylpolyglucosid | - | 10,00 | - | - | ' 10,00 |
| Cocoamidopropylbetain | 2,00 | 2,00 | 5,00 | 2,00 | 2,00 |
| PEG-3-distearat | 2,00 | 2,00 | 2,00 | - | - |
| Verbindung der Formel I* | 1,00 | 1,00 | 2,00 | 0,50 | 0,50 |
| Parfum | 0,50 | 0,50 | 0,50 | 0,50 | 0,50 |
| Citronensäure, Konservierungsmittel | q.s. | q.s. | q.s. | q.s. | q.s. |
| D.C.-Red 33 | 0,15 | 0,10 | 0,10 | 0,10 | 0,15 |
| Acid Yellow 3 | - | 0,08 | 0,05 | - | - |
| FDC-Yellow 6 | - | - | 0,05 | - | - |
| Food Blue 5 | - | 0,08 | - | 0,05 | - |
| Food Yellow 8 | - | - | - | 0,02 | - |
| Wasser | ad 100,00 | ad 100,00 | ad 100,00 | ad 100,00 | ad 100,00 Gew.-% |
| Färbung | Rot | Braun | Rotblond | Rotbraun | Rot |

| | | | | | |
|---|---|---|---|---|---|
| *In den Beispielen 10,12 und 14 ist R¹=R²=Oleyl; R³=CH₃;R⁴=CH₂CH₂OH; x=y=0; Y⁻ =CH₃S0₄⁻. In den Beispielen 11 und 13 ist R¹=R²=C₁₈-C₃₇;R³=CH₃;R⁴=CH₂CH₂OH; x=y=0; Y⁻=Cl⁻. | | | | | |

Die erzielten Färbungen erwiesen sich als wesentlich beständiger als solche, die mit Zusammensetzungen ohne eine Verbindung der Formel I erzielt wurden.

## Patentansprüche

1. Verwendung von 0,5 bis 20 Gew.-% mindestens einer Verbindung der allgemeinen Formel I 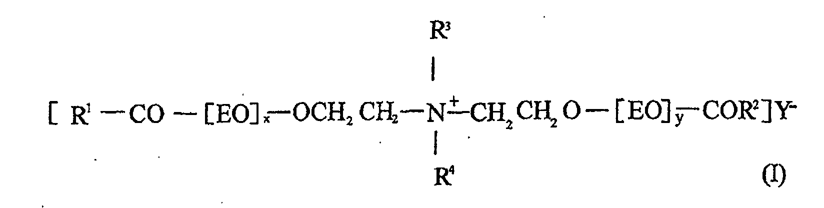 in der R¹ und R² jeweils für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}-H, sowie x,y und z für 0 bis 5, und Y⁻ für ein Anion stehen, zur Abscheidung anionischer Wirkstoffe, ausgewählt aus 0,05 bis 5 Gew.-% anionischen UV-Absorbern und/oder 0,01 bis 2,5 Gew.-% anionischen direktziehenden Haarfarbstoffen, in Haarbehandlungsmitteln auf wäßriger Grundlage auf menschlichem Haar, jeweils berechnet auf die Gesamtzusammensetzung des Mittels.

2. Verwendung eines Gemisches aus 0,5 bis 20 Gew.-%, berechnet auf die Gesamtzusammensetzung.
a) mindestens einer Verbindung der allgemeinen Formel I 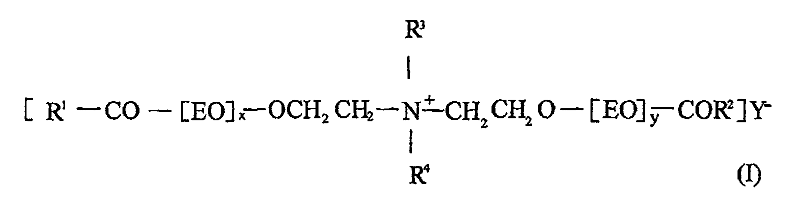 in der R¹ und R² jeweils für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}-H, sowie x,y und z für 0 bis 5, und Y⁻ für ein Anion stehen,
und
b) mindestens einem anionischen UV-Absorber in Haarbehandlungsmitteln auf wäßriger Grundlage zur Erreichung eines stabilen Lichtschutzes.

3. Verwendung eines Gemisches aus 0,5 bis 20 Gew.-%, berechnet auf die Gesamtzusammensetzung
a) mindestens einer Verbindung der allgemeinen Formel I 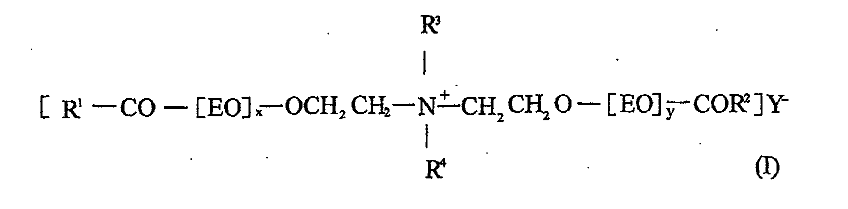 in der R¹ und R² jeweils für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}-H, sowie x,y und z für 0 bis 5, und Y⁻ für ein Anion stehen, und
b) 0,01 bis 2,5 Gew.-% eines anionischen direktziehenden Haarfarbstoffs in Haarbehandlungsmitteln auf wäßriger Grundlage zur Erreichung einer dauerhaften Haarfärbung.

4. Verwendung nach einem der Ansprüche 1 bis 3, **dadurch gekennzeichnet, daß** die Reste R¹ und R² jeweils einen Oleylrest, der Rest R³ eine Methylgruppe, der Rest R⁴ eine Gruppe -CH₂-CH₂-OH, und x und y 0 bedeuten.

5. Verwendung nach einem der Ansprüche 1 bis 3, **dadurch gekennzeichnet, daß** die Reste R¹ und R² jeweils eine C₁₂-C₁₈-Alkylgruppe, der Rest R³ eine Methylgruppe, und der Rest R⁴ eine Gruppe -CH₂-CH₂-O-[EO]_{z}H, und x, y und z 0 bedeuten.

6. Verwendung nach einem oder mehreren der Ansprüche 2 bis 5, wobei als anionischen UV-Absorber mindestens eine Substanz, ausgewählt aus 5-Benzoyl-4-hydroxy-2-methoxybenzolsulfonsäure, 2,2'-Dihydroxy-4,4'-dimethoxy-3,3'-disulfobenzophenon und/oder Phenylbenzimidazolsulfonsäure bzw. deren Natriumsalzen verwendet wird.

7. Verwendung nach einem oder mehreren der Ansprüche 1 bis 6, wobei das Haarbehandlungsmittel 0,1 bis 10 Gew.-% mindestens einer C₁₀-C₂₄-Fettsäure, berechnet auf die Gesamtzusammensetzung, enthält.

## Claims

1. Use of 0.5 to 20 % by weight of at least one compound of the general formula I 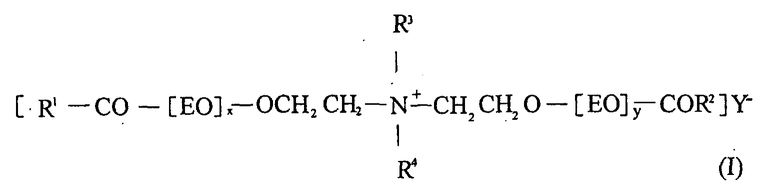 wherein R¹ and R² each are an optionally hydroxy-substituted C₈-C₂₂-alkyl or alkenyl group, R³ and R⁴ are a C₁-C₃-alkyl group or a group -CH₂-CH₂-O-[EO]_{z}-H, x, y and z are 0 to 5, and Y⁻ is an anion, for separation of anionic agents, selected from 0.05 to 0.5 % by weight of anionic UV-absorbers and/or 0.01 to 2.5 % by weight of anionic direct-acting hair dyestuffs in hair treatment compositions on aqueous basis on human hair, calculated to the total composition.

2. Use of a mixture of 0.5 to 20 % by weight, calculated to the total composition of
a) at least one compound of the general formula I 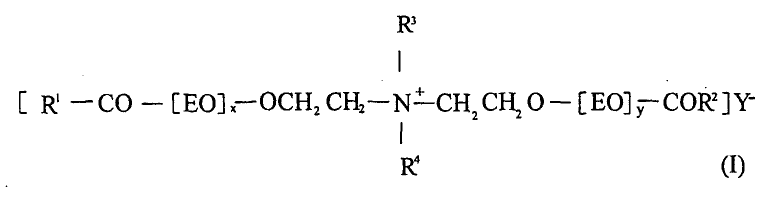 wherein R¹ and R² each are an optionally hydroxy-substituted C₈-C₂₂-alkyl or alkenyl group, R³ and R⁴ are a C₁-C₃-alkyl group or a group -CH₂-CH₂-O-[EO]_{z}-H, x, y and z are 0 to 5, and Y⁻ is an anion, and
b) at least one anionic UV-absorber in hair treatment compositions on aqueous basis to achieve a stable protection against light.

3. Use of a mixture of 0.5 to 20 % by weight, calculated to the total composition of
a) at least one compound of the general formula I 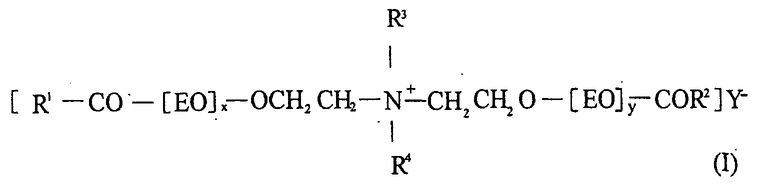 wherein R¹ and R² each are an optionally hydroxy-substituted C₈-C₂₂-alkyl or alkenyl group, R³ and R⁴ are a C₁-C₃-alkyl group or a group -CH₂-CH₂-O-[EO]_{z}-H, x, y and z are 0 to 5, and Y⁻ is an anion, and
b) 0.01 to 2.5 % by weight of an anionic direct-acting dyestuff in hair treatment compositions on aqueous basis to achieve a permanent hair dyeing.

4. Use according to one of claims 1 to 3, wherein the groups R¹ and R² each are an oleyl group, the group R³ is a methyl group, the group R⁴ is a group -CH₂-CH₂-OH, and x and y are 0.

5. Use according to one of claims 1 to 3, wherein the groups R¹ and R² each are a C₁₂-C₁₈-alkyl group, the group R³ is a methyl group, and the group R⁴ is a group -CH₂-CH₂-O-[EO]_{z}H, and y, y and z are 0.

6. Use according to one or more of claims 2 to 5, using as anionic UV-absorber at least one substance selected from 5-benzoyl-4-hydroxy-2-methoxybenzene sulfonic acid, 2.2'-dihydroxy-4.4'dimethoxy-3.3'-disulfobenzophenone and/or phenyl benzimidazole sulfonic acid or the sodium salts thereof.

7. Use according to one or more of claims 1 to 6, whereby the hair treatment composition comprises 0.1 to 10 % by weight of at least one C₁₀-C₂₄-fatty acid, calculated to the total composition.

## Revendications

1. Utilisation de 0,5 à 20 % en poids d'au moins un composé de formule générale I 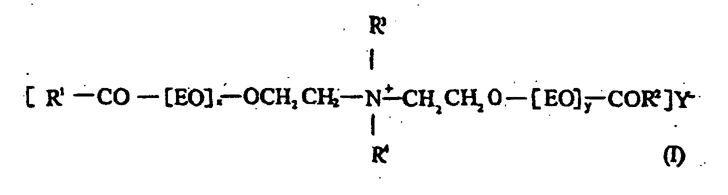 dans laquelle R¹ et R² représentent chacun un groupe alkyle ou alcénylé en C₈ à C₂₂ éventuellement hydroxylé, R³ et R⁴ représentent un groupe alkyle en C₁ à C₃ ou un groupe -CH₂-CH₂-O-[EO]_{z}-H, dans lequel x, y et z représentent 0 à 5 et Y⁻ représente un anion, *pour la préparation* de produits actifs anioniques, sélectionnés parmi des agents anioniques d'absorption des UV à une teneur de 0,05 à 5 % en poids et/ou des colorants anioniques directs des cheveux à une teneur comprise entre 0,01 et 2,5 % en poids, dans des agents de traitement des cheveux à base aqueuse pour les cheveux humains, les pourcentages étant tous calculés par rapport à la composition totale de l'agent.

2. Utilisation d'un mélange à 0,5 à 20 % en poids par rapport à la composition totale,
a) d'au moins un composé de formule générale I 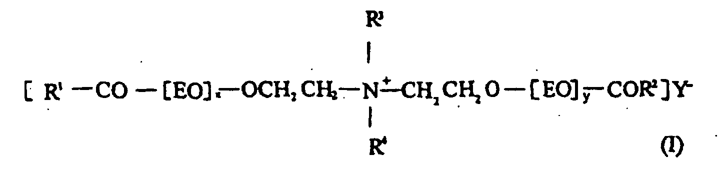 dans laquelle R¹ et R² représentent chacun un groupe alkyle ou alcényle en C₈ à C₂₂ éventuellement hydroxylé, R³ et R⁴ représentent un groupe alkyle en C₁ à C₃ ou un groupe -CH₂-CH₂-O-[EO]_{z}-H, dans lequel x, y et z représentent 0 à 5 et Y⁻ représente un anion, et
b) d'au moins un agent anionique d'absorption des UV, dans des agents de traitement des cheveux à base aqueuse, pour obtenir une protection stable contre la lumière.

3. Utilisation d'un mélange constitué de 0,5 à 20 % en poids par rapport à la composition totale,
a) d'au moins un composé de formule générale I 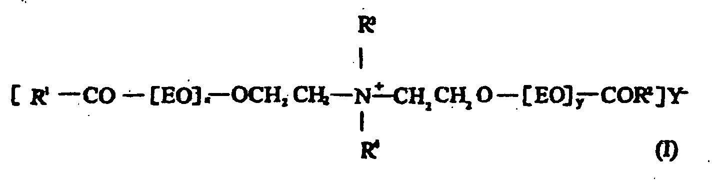 dans laquelle R¹ et R² représentent chacun un groupe alkyle ou alcényle en C₈ à C₂₂ éventuellement hydroxylé, R³ et R⁴ représentent un groupe alkyle en C₁ à C₃ ou un groupe -CH₂-CH₂-O-[EO]_{z}-H, dans lequel x, y et z représentent 0 à 5 et Y⁻ représente un anion, et
b) de 0,01 à 2,5 % en poids d'un colorant anionique direct des cheveux, dans des agents de traitement des cheveux à base aqueuse, pour obtenir une coloration durable des cheveux.

4. Utilisation selon l'une des revendications 1 à 3, **caractérisée en ce que** les radicaux R¹ et R² représentent chacun un radical oléyle, le radical R³ un groupe méthyle, le radical R⁴ un groupe -CH₂-CH₂-OH, et x et y représentent 0.

5. Utilisation selon l'une des revendications 1 à 3, **caractérisée en ce que** les radicaux R¹ et R² représentent chacun un groupe alkyle en C₁₂ à C₁₈, le radical R³ un groupe méthyle et le radical R⁴ un groupe -CH₂-CH₂-O-[EO]_{z}H, et x et y représentent 0.

6. Utilisation selon l'une ou plusieurs des revendications 2 à 5, dans laquelle on utilise, comme agent anionique d'absorption des UV, au moins une substance sélectionnée parmi l'acide 5-benzoyl-4-hydroxy-2-méthoxybenzènsulfonique, la 2,2'-dihydroxy-4,4'-diméthoxy-3,3'-disulfobenzophénone et/ou l'acide phénylbenzimidazole sulfonique ou leurs sels de sodium.

7. Utilisation selon l'une ou plusieurs des revendications 1 à 6, dans laquelle l'agent de traitement des cheveux contient de 0,1 à 10 % en poids d'au moins un acide gras en C₁₀ à C₂₄, le pourcentage étant calculé par rapport à la composition totale.
